# DEMANDE DE BREVET EUROPEEN

(11) **EP 0 919 200 A1**
(43) Date de publication de la demande: **02.06.1999**
(21) Numéro de dépôt: 97402795.5
(22) Date de dépôt: 20.11.1997
(51) Int. Cl.: A61B 17/76

(54) **Systéme pour ostéosynthèse de l'extrémité supérieure du fémur**

(71) Demandeur: VM Tech, 94230 Cachan (FR)
(72) Inventeur: de la Caffiniere, Jean-Yves, 75005 Paris (FR)
(74) Mandataire: Debay, Yves

(57) **Abrégé**

La présente invention concerne un système pour ostéosynthèse de l'extrémité supérieure du fémur caractérisé en ce qu'il comporte un disposif pour ostéosynthèse à deux clous (10,20), dont un clou (20) cervicocéphalique et un dispositif de visée destiné à la mise en place d'une vis (26) transfixiante dans le col du fémur et du clou (20) annoncé.

## Description

L'invention concerne un système pour ostéosynthèse de l'extrémité supérieure du fémur, conçu particulièrement pour les fractures instables par et sous trochantériennes.

Ces systèmes doivent posséder des caractéristiques mécaniques suffisantes pour résister aux charges d'utilisation, avoir une mise en place préservant les parties molles ainsi qu'un ancrage optimal dans le col du fémur, afin d'éviter une effraction articulaire, c'est-à-dire d'éviter que les éléments des systèmes n'endommagent la tête du fémur.

L'un des systèmes existant est un système vis-plaque. Ce système possède un ancrage satisfaisant dans la tête fémorale, par contre sa mise en place peut générer un raccourcissement du membre inférieur qui n'est pas souhaitable.

L'autre système est un système clou-plaque. Ce système permet un glissement de la pièce osseuse cervicocéphalique sur le clou qui améliore la reconstitution osseuse. Par contre, ce glissement peut provoquer, sur des anatomies particulières, par exemple une faible densité osseuse, une effraction articulaire qui peut se révéler dangereuse pour le patient.

La présente invention a pour objet de pallier les inconvénients de ces systèmes en proposant dans un premier but un système ayant une mise en place préservant les partie molles, et respectivement dans un second but un dispositif pour ostéosynthèse de l'extrémité supérieure du fémur conjuguant un ancrage optimal dans le col du fémur, minimisant le risque d'effraction articulaire.

Ce premier but est atteint par le fait que le système pour ostéosynthèse de l'extrémité supérieure du fémur comporte un dispositif à deux clous dont un clou cervicocéphalique et un dispositif de visée destiné à la mise en place d'une vis transfixiante dans le col du fémur et le clou annoncé

Ce second but est atteint but est atteint par le fait que le dispositif pour ostéosynthèse de l'extrémité supérieure du fémur, comprend un clou centromédullaire, et un second clou cervicocéphalique enchevillés l'un dans l'autre au niveau de la région trochantérienne, ces deux clous étant maintenus solidaires entre eux, d'une part, par des moyens de blocage en translation, et d'autre part, par des moyens de blocage en rotation, le clou centromédullaire étant fixé au fémur par des moyens de solidarisation et le clou cervicocéphalique étant ancré dans le col du fémur par une vis transfixiante.

Selon une autre caractéristique de l'invention, l'orientation des moyens de blocage en rotation et en translation du clou cervicocéphalique est repérée sur ce dernier et sur le dispositif de visée associé.

Selon une autre caractéristique de l'invention, le clou centromédullaire est, coudé selon un angle déterminé, de longueur déterminée, et destiné à être introduit dans le canal médullaire de l'extrémité supérieure du fémur, il comporte, dans sa partie supérieure une lumière transversale, dont l'axe fait un angle déterminé avec l'axe de la partie supérieure du clou centromédullaire pour après mise en place être confondu avec l'axe du col du fémur et de dimension déterminée, et dans sa partie inférieure des moyens de solidarisation sur le fémur.

Selon une autre caractéristique de l'invention, la longueur du clou centromédullaire est comprise entre 19 et 45 cm.

Selon une autre caractéristique de l'invention, le clou cervicocéphalique, est de longueur déterminée, de largeur maximale inférieure à la dimension de la lumière transversale pratiquée dans le clou centromédullaire, et destiné à être introduit dans l'axe du col du fémur, et comporte des moyens de solidarisation à l'extrémité du fémur.

Selon une autre caractéristique de l'invention, la longueur du clou cervicocéphalique est comprise entre 7 et 11 cm.

Selon une autre caractéristique de l'invention, les moyens de blocage en translation, et les moyens de blocage en rotation qui solidarisent les deux clous cervicocéphalique et centromédullaire sont confondus.

Selon une autre caractéristique de l'invention, le moyen unique de solidarisation des deux clous cervicocéphalique et centromédullaire est une clavette qui traverse d'un part, le clou centromédullaire par un canal axial et d'autre part, le clou cervicocéphalique part une lumière transversale faisant un angle déterminé avec l'axe longitudinal dudit clou.

Selon une autre caractéristique de l'invention, le moyen unique de solidarisation des deux clous cervicocéphalique et centromédullaire est une vis qui traverse le clou centromédullaire par un canal axial et prend appui sur un méplat pratiqué sur la surface supérieure du clou cervicocéphalique.

Selon une autre caractéristique de l'invention, le moyen unique de solidarisation des deux clous cervicocéphalique et centromédullaire est une vis qui traverse le clou centromédullaire dans sa partie supérieure par un canal axial et vient se loger dans une cavité pratiquée sur le clou cervicocéphalique, la forme de la cavité est telle qu'il peut exister une approximation de la position du clou cervicocéphalique lors de la mise en plas de la vis.

Selon une autre caractéristique de l'invention, le moyen de blocage en translation du clou cervicocéphalique est une vis qui prend appui sur ledit clou.

Selon une autre caractéristique de l'invention, le moyen de blocage en rotation du clou cervicocéphalique est constitué par le choix d'une forme non circulaire déterminée, pour la section du clou cervicocéphalique et pour la lumière transversale de la partie supérieure du clou centromédullaire.

Selon une autre caractéristique de l'invention, les moyens de solidarisation du clou centromédullaire au fémur sont des vis corticales de verrouillage diaphysaire qui traversent le clou perpendiculairement à son axe longitudinal.

Selon une autre caractéristique de l'invention, l'angle entre l'axe de la lumière transversale de la partie supérieure du clou centromédullaire et l'axe vertical est compris entre 125 et 135°.

Un troisième but est un dispositif de visée facilitant la mise en place du dispositif pour l'ostéosynthèse de l'extrémité supérieure du fémur.

Selon ce but de l'invention, le dispositif de visée pour la mise en place d'un dispositif pour ostéosynthèse comporte des moyens de fixation au clou centromédullaire, des moyens de visée pour la mise en place du clou cervicocéphalique, des moyens de repérage pour un positionnement optimal, des moyens de blocage en rotation et en translation du clou cervicocéphalique, des moyens de visée pour la mise en place des moyens de solidarisation des clous centromédullaire et cervicocéphalique avec fémur.

Selon une autre caractéristique de l'invention, le dispositif de visée pour la mise en place du dispositif pour ostéosynthèse comprend deux branches fixées entre elles de façon amovible, formant un angle déterminé, une première branche parallèle au clou cervicocéphalique, de longueur déterminée en fonction du patient, comprenant des moyens de solidarisation au clou centromédullaire, des moyens de visée pour la mise en place des moyens de fixation du clou cervicocéphalique sur le fémur, et une deuxième branche, parallèle à la partie inférieure du clou centromédullaire, de longueur déterminée en fonction du patient, comportant des moyens de visée pour la mise en place du clou cervicocéphalique dans l'axe du col du fémur, des moyens de visée pour la mise en place des moyens de fixation du clou centromédullaire sur le fémur, et des moyens de repérage pour un positionnement optimal des moyens de blocage en rotation et en translation du clou cervicocéphalique.

Selon une autre caractéristique de l'invention, le dispositif de visée pour la mise en place du dispositif pour ostéosynthèse comprend une barre coudée, la première section inférieure au coude est verticale et comprend des moyens de visée pour la mise en place du clou cervicocéphalique dans l'axe du col du fémur, des moyens de visée pour la mise en place des moyens de fixation du clou centromédullaire sur le fémur, et des moyens de repérage pour un positionnement optimal des moyens de blocage en rotation et en translation du clou cervicocéphalique, la deuxième section étant sensiblement parallèle au clou cervicocéphalique lorsque celui-ci est en place dans le col de fémur et comprend des moyens de solidarisation au clou centromédullaire, des moyens de visée pour la mise en place des moyens de fixation du clou cervicocéphalique sur le fémur.

Selon une autre caractéristique de l'invention, les moyens de solidarisation du dispositif de visée au clou centromédullaire comprennent une douille (60), une lumière (56) pratiquée dans la partie supérieure du système de visée, et un moyen (55) de fixation, de type boulon passant par la lumière (56), traversant la douille (60) pour coopérer avec le taraudage (14) du clou (10) centromédullaire.

Selon une autre caractéristique de l'invention, les moyens de solidarisation, comprennent une pièce (100) en L, fixée par sa première branche (101), parallèle à la section (92) supérieure du système de visée, sur cette même section (92), grâce à des moyens de fixation (102), au clou (10) centromédullaire et par sa deuxième branche (103), comprenant à son extrémité, libre une pièce (105) cylindrique creuse, sur le clou (10) centromédullaire, par l'intermédiaire d'un moyen de fixation qui traverse la pièce (105) cylindrique creuse.

Selon une autre caractéristique de l'invention, les moyens pour la mise en place du clou cervicocéphalique sont une lumière transversale faisant un angle déterminé avec l'axe longitudinal de la branche supérieure du système de visée.

Selon une autre caractéristique de l'invention, l'angle entre la lumière transversale du système de visée et l'axe longitudinal de la branche inférieure du système de visée est le même que l'angle entre l'axe de la lumière transversale de la partie supérieure du clou centromédullaire et l'axe longitudinal de cette dite partie supérieure.

Selon une autre caractéristique de l'invention, la surface extérieure de la branche supérieure du système de visée comporte au niveau de la lumière transversale, un repère visualisant l'orientation, que devront prendre les moyens de blocage en rotation et en translation, du clou cervicocéphalique, lors de la mise en place.

Selon une autre caractéristique de l'invention, que les moyens de visée pour les mises en place des moyen de fixation des deux clous centromédullaire et cervicocéphalique, avec le fémur sont des lumières perpendiculaires aux axes longitudinaux des branches correspondantes, du dispositif de visée.

D'autres particularités et avantages de la présente invention apparaîtront plus clairement à la lecture de la description ci-après, faite en référence aux dessins annexés dans lesquels :
- la figure 1 représente une vue en coupe du dispositif pour ostéosynthèse,
- la figure 2 représente une vue en coupe d'une variante de fabrication du dispositif pour ostéosynthèse,
- les figures 3A et 3B représentent une vue en coupe du dispositif de visée pour la mise en place du dispositif pour ostéosynthèse, ainsi qu'une vue détaillée de l'emplacement des moyens de repérage de l'orientation des moyens de blocage en rotation et en translation de clou cervicodéphalique,
- les figures 4A, 4B, et 4 C représentent une vue en coupe transversale du clou cervicocéphalique du dispositif pour ostéosynthèse, pour les différentes variantes de fabrication.
- la figure 5 représente une vue en coupe du dispositif pour ostéosynthèse après sa est mise en place dans l'extrémité supérieure du fémur grâce au système de l'invention.
- la figure 6 représente une vue en coupe du système d'ostéosynthèse de l'extrémité supérieure du fémur, lorsque le dispositif de visée est fixé sur le clou centromédullaire.
- la figure 7 représente une vue en coupe du détail d'une variante de fabrication pour le moyen unique de blocage en rotation et en translation des deux clous, centromédulaire et cervicocéphalique, du dispositif d'ostéosynthèse.
- la figure 8 représente une vue en coupe du système d'ostéosynthèse de l'extrémité supérieure du fémur, lorsque le dispositif de visée est fixé sur le clou centromédullaire pour une variante de fabrication.

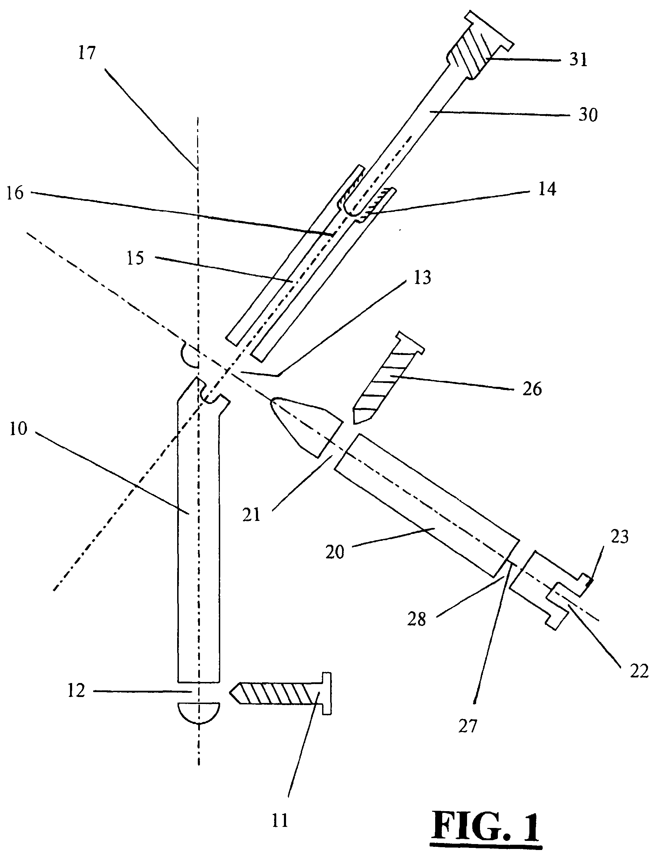

La figure 1 représente une vue en coupe du dispositif pour ostéosynthèse. Il apparaît sur cette figure que le dispositif comprend principalement deux clous (10,20), le clou (10) centromédullaire et le clou (20) cervicocéphalique, et une clavette (30). Le clou centromédullaire (10) est constitué d'une barre coudée de section cylindrique et de longueur déterminée en fonction du patient. L'angle d'inclinaison par rapport à l'axe vertical (17) est conforme à l'angulation naturelle du canal médullaire de l'extrémité supérieure du fémur et est choisi entre 45 et 55°. La longueur est choisie de préférence entre 15 et 30 cm selon la morphologie du patient. La partie supérieure du coude comporte d'une part, sur toute la longueur de cette partie, un canal (15) axial de diamètre déterminé dont l'extrémité supérieure comporte un taraudage (14), et d'autre part une première lumière (13) transversale de dimension déterminée et dont l'axe longitudinal (27) fait un angle déterminé avec l'axe vertical (17) et se trouve à une distance donnée de l'extrémité supérieure du clou (10) centromédullaire, correspondant aux morphologies des patients. Cet angle est choisi entre 125 et 135°. La partie inférieure du coude est sensiblement verticale. L'extrémité inférieure de cette partie est constituée d'un tronc de cône arrondi. Elle comporte également, au moins une lumière (12) de diamètre déterminé, perpendiculaire à son axe longitudinal, et destinée à recevoir une première vis (11). Il est à la portée de l'homme du métier de rajouter une ou plusieurs autres lumières (12), ces lumières (12) étant destinées à assurer la solidarisation du clou centromédullaire (10) avec le fémur par l'intermédiaire de vis (11) corticales de verrouillage diaphysaire. Le clou (10) centromédullaire est destiné à être introduit dans le canal médullaire de l'extrémité du fémur par le sommet du trochanter major. Le clou (20) cervicocéphalique est constitué d'une barre de section déterminée et de longueur déterminée. La longueur est choisie par exemple entre 7 et 10 cm selon la morphologie du patient. L'extrémité supérieure de ce clou (20) est constituée d'un tronc de cône arrondi. Cette extrémité comporte également une première lumière (21) transversale, perpendiculaire à l'axe (27) longitudinale du clou (20) cervicocéphalique, destinée à recevoir une vis (26). Il est à la portée de l'homme du métier de rajouter une ou plusieurs autres lumières (21), ces lumières (21) étant destinées à assurer la solidarisation du clou (20) cervicocéphalique avec l'extrémité supérieure du fémur, par l'intermédiaire de vis (26) transfixiantes. L'extrémité inférieure du clou (20) cervicocéphalique comporte, une seconde lumière (28) transversale perpendiculaire à l'axe longitudinal dudit clou (20) et de diamètre déterminé, et un canal (22) sensiblement cylindrique, suivant l'axe longitudinal (27) du même clou (20), destiné à recevoir du matériel ancillaire spécifique. La face extérieure de cette extrémité comporte un repère (23) qui visualise la direction de l'axe de la second lumière (28) la plus proche du repère (23). Ce repère (23) peut être matérialisé, par exemple, par une encoche, un ergot ou un poinçon. Le clou (20) cervicocéphalique est destiné à être introduit dans l'axe du col du fémur. La clavette (30) est de dimension semblable au canal (15) axial situé dans la partie supérieure au coude du clou (10) centromédullaire et comporte un filetage (31) à son extrémité supérieure.

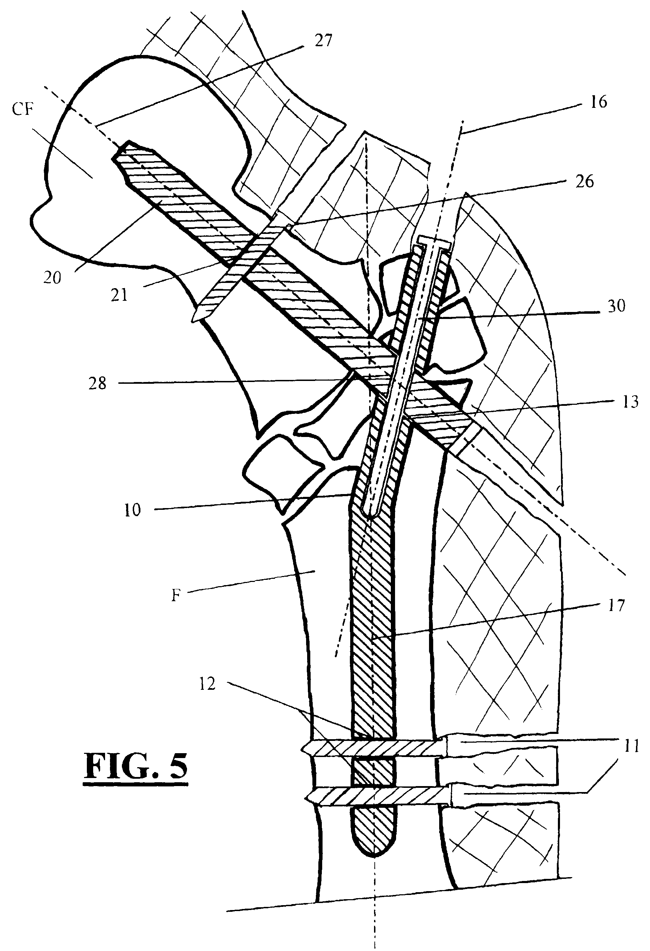

La figure 5 représente une vue en coupe du dispositif pour ostéosynthèse, lorsqu'il est en place dans l'extrémité du fémur. Ainsi lorsque la clavette (30) est visée dans la partie supérieure du clou (10) centromédullaire, elle passe par le canal (15) axial de la partie supérieure du coude du clou (10) centromédullaire, en traversant le clou (20) cervicocéphalique par la seconde lumière (28) située la plus proche de son extrémité inférieure, bloquant ainsi en rotation et en translation les deux clous l'un par rapport à l'autre.

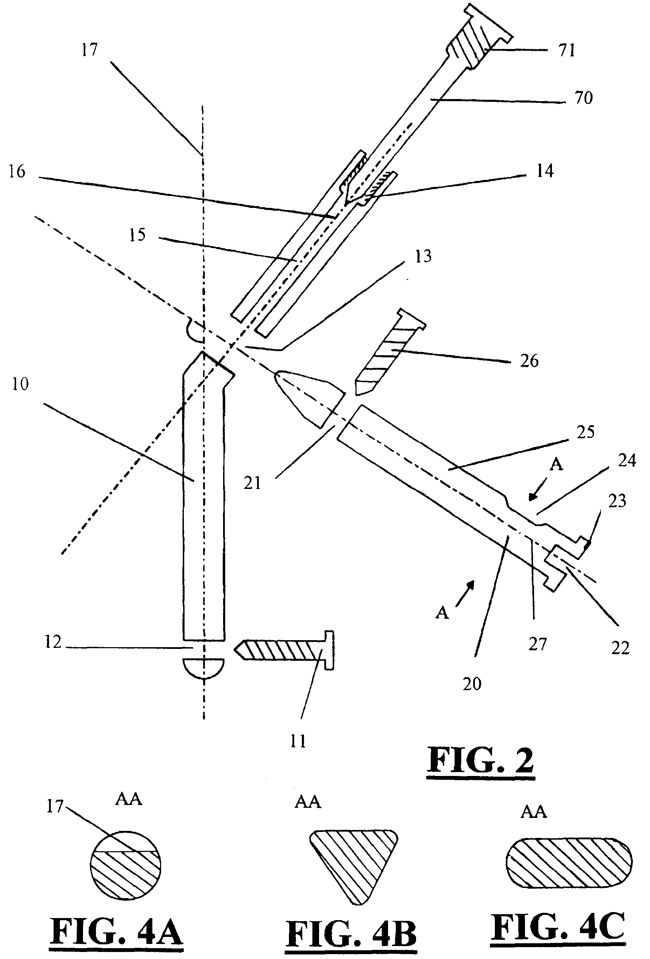

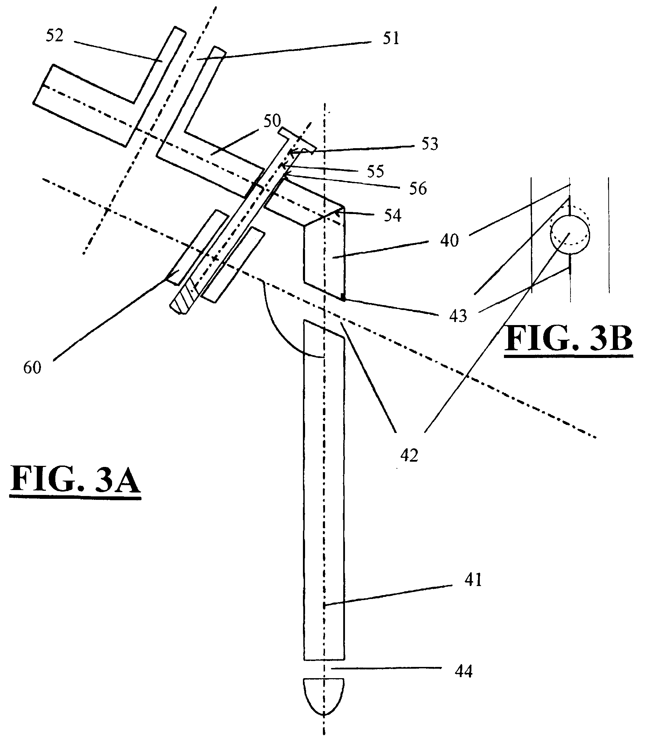

La figure 2 représente une vue en coupe d'une variante de fabrication du dispositif pour ostéosynthèse. Cette variante diffère de la figure 1 par le fait que l'on remplace la clavette (30) par une vis-pointeau (70), et que la seconde lumière (28) transversale située la plus proche de l'extrémité inférieure du clou (20) cervicocéphalique est remplacée en lieu et place, par un méplat (24) situé sur la face supérieure du clou (20) cervicocéphalique, le reste du dispositif restant identique. Lors de la mise en place du dispositif pour ostéosynthèse la vis-pointeau (70) passe par le canal (15) axial de la partie supérieure du coude du chou (20) centromédullaire et lors du serrage finale, la vis-pointeau (70) vient en appui sur le méplat (24) bloquant ainsi en rotation et en translation les deux clous l'un par rapport à l'autre.

Les figures 4A, 4B, et 4C représentent une vue en coupe transversale selon le plan AA du clou cervicocéphalique du dispositif pour ostéosynthèse F, pour les différentes variantes de fabrication. La figure 4A est la coupe transversale selon le plan AA du clou cervicocéphalique du dispositif pour ostéosynthèse pour la variante décrite à la figure 2. Les figures 4B et 4C représentent une vue en coupe transversale selon le plan AA du clou cervicocéphalique du dispositif pour ostéosynthèse, pour des variantes de fabrication qui consistent en fait à dissocier les moyens de blocage en rotation et les moyens de blocage en translation des deux clous l'un par rapport à l'autre. Dans ces 2 variantes de fabrication, le moyen de blocage en translation est une vis-pointeau (70) telle que décrite précédemment dans la figure 2. Le moyen de blocage en rotation consiste en un choix d'une forme particulière pour la section transversale du clou (20) cervicocéphalique et de la lumière (13) transversale située dans la partie supérieure du coude du clou (10) centromédullaire. Le choix peut se faire parmi des formes non circulaires déterminées, pour interdire ainsi la rotation du clou (20) cervicocéphalique dans la lumière (13) transversale du clou (10) centromédullaire. Les figures 4A et 4B donnent deux exemples de forme parmi d'autres possibles, pour empêcher la rotation. La première forme est un triangle isocèle dont les sommets sont arrondits. La deuxième forme est une ellipse aplatie.

La figure 3 représente une vue en coupe du dispositif de visée pour la mise en place du dispositif pour ostéosynthèse et formant le système le d'ostéosynthèse. Le dispositif de visée comprend deux branches, une branche (50) supérieure et une branche (40) inférieure, faisant entre elles un angle déterminé par l'intermédiaire d'un biseau (54), et une douille (60) servant à la fixation du dispositif de visée sur le clou (10) centromédullaire. La branche supérieure (50) est une barre de section déterminée comportant dans sa partie supérieure, une première lumière (51) transversale, perpendiculaire à l'axe longitudinal de la branche supérieure (50). Cette première lumière (51) est prolongée vers le haut par un tube (52) de diamètre intérieur sensiblement égale au diamètre de ladite lumière (51). Cette première lumière (21) et ce tube (52) permettent de guider les moyens ancillaires spécifiques pour positionner correctement une vis (26) de solidarisation du clou (20) cervicocéphalique avec le col du fémur. Cette vis (26) venant ce loger dans la deuxième lumière (21 fig. 1 et 2) de la partie supérieure du clou (20, fig. 1 et 2) cervicocéphalique. Dans sa partie inférieure, la branche supérieure (50) comporte une deuxième lumière (56) transversale, dont l'axe (53) de symétrie est confondu avec l'axe (16, fig. et 2) du canal (15, fig. 1 et 2) axial de la partie supérieure au coude du clou (10, fig. 1 et 2) centromédullaire, lorsque le dispositif de visé est monté sur ledit clou (10 fig. 1 et 2). Le diamètre de cette deuxième lumière (56) permet le passage d'un moyen de fixation (55), de type boulon qui vient se fixer dans le taraudage (14, fig. 1 et 2) du clou (10, fig. 1 et 2) centromédullaire. L'angle du biseau (54) entre les deux branches (50,40) du dispositif de visée est déterminé pour, après fixation du disposition sur le clou (10) centromédullaire, que la branche inférieure (40) du dispositif de visée soit sensiblement verticale et que la branche supérieure soit parallèle à l'axe (27, fig. 1 et 2) de la lumière (13, fig. 1 et 2) transversale de la partie supérieure du coude du clou (10, fig. 1 et 2) centromédullaire. La branche inférieure (40) comporte, dans sa partie basse, au moins une lumière (44) transversale de visée, perpendiculaire à son axe longitudinal (41), et dans sa partie supérieure une deuxième lumière (42) transversale de visée dont l'axe fait un angle déterminé avec l'axe verticale (41). La valeur de cet angle est identique à la valeur de l'angle entre l'axe longitudinal de la lumière (13, fig. 1 et 2) du clou (10, fig. 1 et 2) centromédullaire. La douille (60) de fixation est un tube de longueur déterminée pour permettre le maintien du dispositif de visée à proximité de la peau,. Cette douille (60) à un diamètre intérieur qui permet le passage d'un moyen de fixation (55) , de type boulon, dont la tête appuie sur la branche supérieure (50) du dispositif de visée et le filetage (14, fig. 1 et 2) de la partie supérieure du clou (10, fig. 1 et 2) centromédullaire.

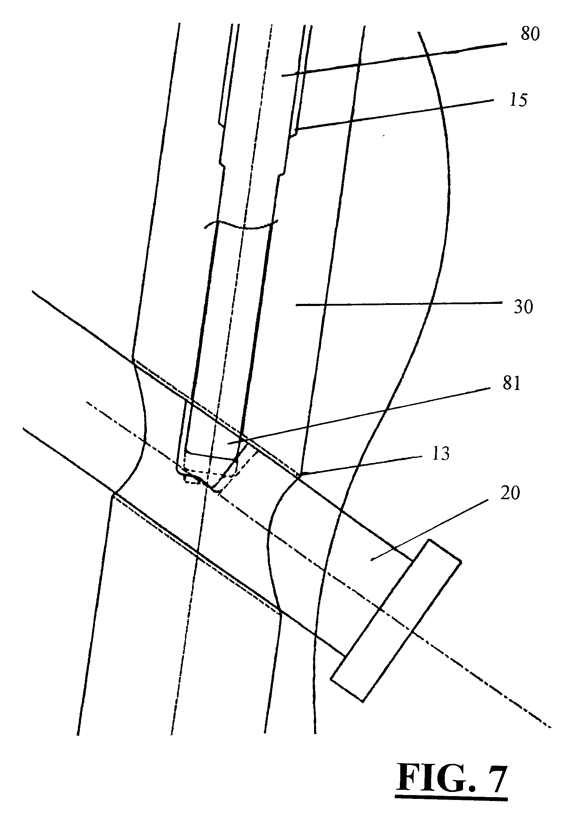

La figure 7 représente une vue en coupe du détail d'une variante de fabrication pour le moyen unique de blocage en rotation et en translation deux clous centromédullaire et cervicocéphalique du dispositif d'ostéosynthèse. Cette variante diffère de la figure 1 par le fait que l'on remplace la clavette (30 fig 1) par une vis (80) plus courte, et que la seconde lumière (28) transversale située la plus proche de l'extrémité inférieure du clou (20) cervicocéphalique est remplacée en lieu et place, par une cavité (29) en tronc de cône de dimension semblable aux dimensions de l'extrémité inférieure (81) de la vis (80)) et située sur la face supérieure du clou (20) cervicocéphalique, le fond la cavité (29) comportant une surface horizontale et une surface inclinée. Le reste du dispositif reste identique. Lors de la mise en place du dispositif pour ostéosynthèse, la vis (80) passe par le canal (15) axial de la partie supérieure du coude du clou (20) centromédullaire et l'extrémité inférieure (81) de la vis (80) vient se loger dans la cavité (29), l'extrémité inférieure (81) de la clavette venant en appui sur la surface horizontale du fond de la cavité (29). La forme de tronc de cône de la cavité (29) améliore le placement de l'extrémité inférieure (81) de la vis (80) dans la cavité (29). Lors de la mise en place de la vis (80), si celle-ci n'est pas parfaitement logée dans la cavité (29), l'un des coins de l'extrémité inférieure (81) de la vis (80) est alors en appui sur la surface inclinée du tronc de cône de la cavité (29). Il suffit du vissage finale pour que le coin de l'extrémité inférieure (81) de la vis (80) en appui sur la surface inclinée du tronc de la cavité (29) glisse sur cette surface en exerçant une traction sur le clou (20) cervicocéphalique jusqu'à ce que la vis (80) soit parfaitement logée dans la cavité (29). On obtient ainsi une solidarisation optimal des clous (10,20) centromédulaire et cervicocéphalique. Les pointillés montrent sur la figure 7 le mouvement de glissement du coin de l'extrémité inférieure (81) de la vis (80) sur la surface inclinée de la cavité (29).

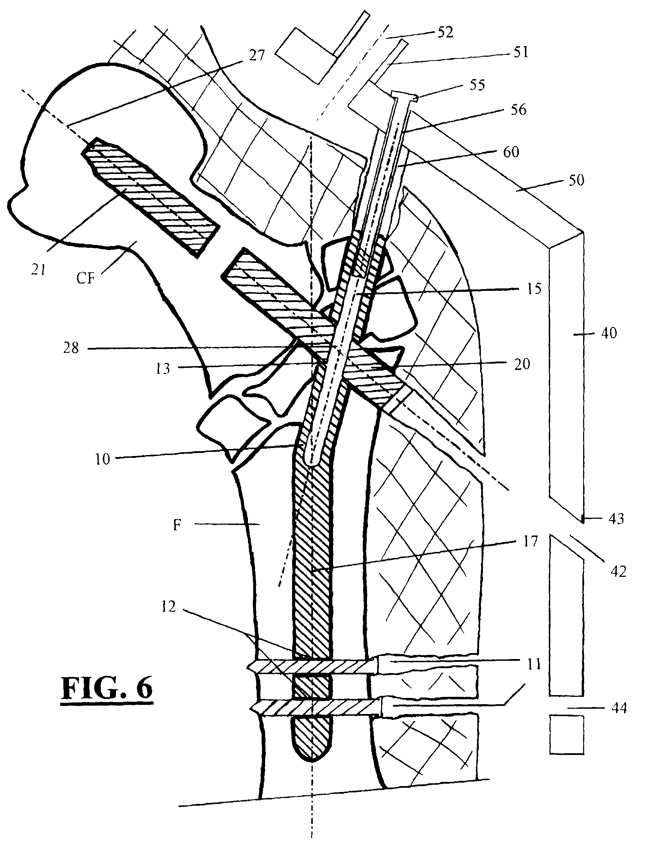

La figure 6 représente une vue en coupe du système d'ostéosynthèse de l'extrémité supérieure du fémur, lorsque le dispositif de visée est fixé sur le clou centromédullaire. La mise en place du dispositif de visée sur le clou (15) centromédullaire s'effectue de la façon suivante, une fois le clou (10) centromédullaire en place dans le canal médullaire du fémur F, une douille (60) de fixation est placée sur l'extrémité supérieure du clou (10) centromédullaire de telle sorte que l'axe longitudinal de la douille (60) soit confondu avec l'axe (16) longitudinal de la partie supérieure au coude du clou (10) centromédullaire. Le dispositif de visée est placé ensuite sur l'extrémité supérieure de la douille (60). La solidarisation du tout est obtenu par un moyen (55) de fixation de type boulon, traversant le dispositif de visée par la deuxième lumière (56) de la branche supérieure (50) et la douille (60) suivant son axe longitudinal, vissé sur le taraudage (14) du clou (10) centromédullaire. Dans cette configuration, le système de visée est situé à l'extérieur du corps du patient, préservant ainsi les parties molles lors de l'introduction des différents éléments du dispositif pour l'ostéosynthèse de l'extrémité supérieure du fémur, à savoir le clou (20) cervicocéphalique introduit dans l'axe du col du fémur CF, la vis (26 fig. 1 et 2) transfixiante qui solidarise le clou (20) cervicocéphalique avec le col du fémur et la ou les vis (11) corticales de verrouillage diaphysaire qui solidarise le clou (10) centromédullaire avec le fémur F. Pour un positionnement optimale des moyens de blocage en rotation et en rotation du clou (20) cervicocéphalique, un repère (43) est disposé sur la surface extérieure du dispositif de visée. Ainsi l'utilisateur peut positionner de façon adéquate ces moyens de blocage en alignant le repère (43) du dispositif de visée et le repère (23) du clou (20) cervicocéphalique.

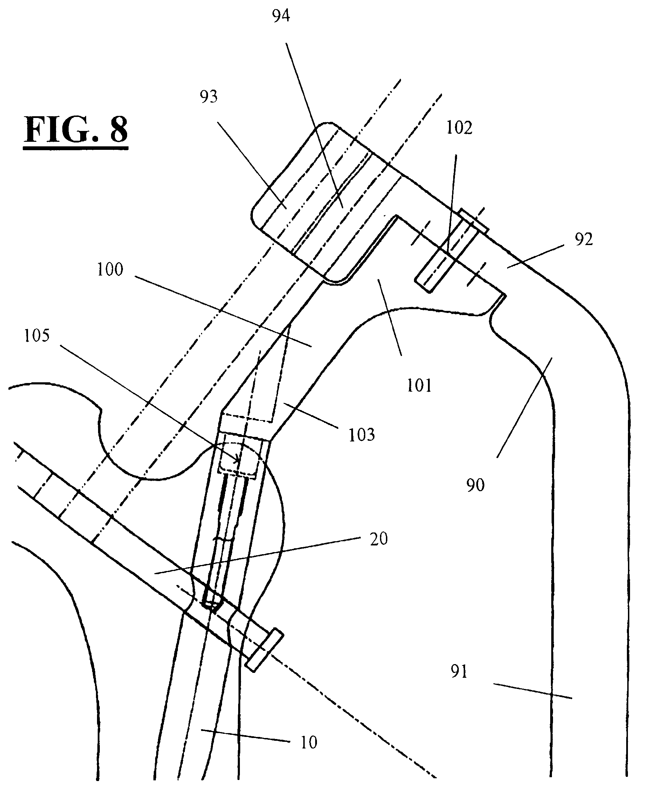

La figure 8 représente une vue en coupe du système d'ostéosynthèse de l'extrémité supérieure du fémur, lorsque le dispositif de visée est fixé sur le clou centromédullaire pour une variante de fabrication. Dans cette variante le dispositif de visée est constitué d'une barre coudé (90). La première section (91), inférieure au coude est verticale et sensiblement identique à la branche (40) inférieure de la variante de fabrication décrite par les figures 3A et 3B. La deuxième section (92) est parallèle au clou (20) cervicocéphalique lorsque celui-ci est en place dans le col du fémur. L'extrémité de cette deuxième section comporte les moyens de (93,94) visée pour la mise en place des moyens (26) de solidarisation du clou (20) cervicocéphalique, avec le col du fémur. Ces moyens comprennent deux canons (93,94) juxtaposés dirigés vers le col du fémur et perpendiculaires à la deuxième branches (92). Ces canons (93,94) ont la même fonction que la première lumière (51) transversale et le tube (51), perpendiculaire à l'axe longitudinal de la branche supérieure (50) du dispositif de visée de la variante décrite aux figures 3A et 3B mais permettent de choisir la position idéale de la vis (26) transfixiante qui va solidariser le clou (20) cervicocéphalique avec le col du fémur. La deuxième section comprend également sur la surface dirigée vers le col du fémur une cavité (95). Les dimensions de cette cavité (95) sont telles que la deuxième section (92) peut recevoir les moyens (100) de fixation du dispositif de visée sur le clou (10) centromédullaire. Ses moyens comprennent une pièce (100) en L, la première branche (101) de cette pièce (100) est parallèle à la branche (92) supérieure du dispositif de visée et fixée sur celle-ci par des moyens de fixation(102), par exemple, une vis. La deuxième branche (103) comporte à son extrémité libre une pièce (105) cylindrique et creuse. Cette pièce (105) a des dimensions qui permettent de l'introduire à l'extrémité de la branche supérieure du clou (10) centromédullaire. La solidarisation finale de la pièce (100) en L avec le clou (10) centromédullaire est obtenue grâce à un moyen de fixation qui traverse la pièce (105) cylindrique et se visse sur le taraudage (14) du clou (10) centromedullaire. Cette variante de fabrication du dispositif de visée permet de mettre en place la clavette (90) sans retirer le système de visée.

D'autres modifications à la portée de l'homme de métier font également partie de l'esprit de l'invention.

## Revendications

1. Système pour ostéosynthèse de l'extrémité supérieure du fémur caractérisé en ce qu'il comporte un dispositif pour ostéosynthèse à deux clous (10,20), dont un clou (20) cervicocéphalique et un dispositif de visée destiné à la mise en place d'une vis (26) transfixiante dans le col du fémur et du clou (20) annoncé.

2. Système pour ostéosynthèse de l'extrémité supérieure du fémur selon la revendication 1, caractérisé en ce que le dispositif pour ostéosynthèse comprend un clou (10) centromédullaire, et un clou (20) cervicocéphalique enchevillés l'un dans l'autre au niveau de la région trochantérienne, ces deux clous (10,20) étant maintenus solidaires entre eux, d'une part, par des moyens de blocage en translation, et d'autre part, par des moyens de blocage en rotation, le clou (10) centromédullaire étant fixé au fémur par des moyens de solidarisation (11) et le clou (20) cervicocéphalique étant ancré dans le col du fémur par une vis (26) transfixiante.

3. Système pour ostéosynthèse de l'extrémité supérieure du fémur selon la revendication 1 ou 2, caractérisé en ce que l'orientation des moyens de blocage en rotation et en translation (30,28,24,70), du clou (20) cervicocéphalique est repérée sur ce dernier et sur le dispositif de visée.

4. Système pour ostéosynthèse de l'extrémité supérieure du fémur selon l'une des revendication 1 à 3, caractérisé en ce que le clou (10) centromédullaire est, coudé selon un angle déterminé, de longueur déterminée, et destiné à être introduit dans le canal médullaire de l'extrémité supérieure du fémur, comportant dans sa partie supérieure une lumière (13) transversale, dont l'axe de symétrie fait un angle déterminé avec l'axe vertical (17), pour, après mise en place, être confondu avec l'axe du col du fémur, et de dimension déterminée, et dans sa partie inférieure des moyens de solidarisation (12,11) sur le fémur.

5. Système pour ostéosynthèse de l'extrémité supérieure du fémur selon la revendication 4, caractérisé en ce que la longueur du clou (10) centromédullaire est comprise entre 19 et 45 cm.

6. Système pour ostéosynthèse de l'extrémité supérieure du fémur selon l'une des revendications 1 à 5, caractérisé en ce que le clou (20) cervicocéphalique, est de longueur déterminée, de largeur maximale inférieure à la dimension de la lumière (28) transversale pratiquée dans le clou (13) centromédullaire, destiné à être introduit dans l'axe du col du fémur, et comporte des moyens de solidarisation (26,21) à l'extrémité du fémur.

7. Système pour ostéosynthèse de l'extrémité supérieure du fémur selon l'une des revendications 6, caractérisé en ce que la longueur du clou (20) cervicocéphalique est comprise entre 7 et 11 cm.

8. Système pour ostéosynthèse de l'extrémité supérieure du fémur selon l'une des revendications 1 à 7, caractérisé en ce que les moyens de blocage en translation, et les moyens de blocage en rotation qui solidarisent les deux clous (10,20) cervicocéphalique et centromédullaire sont confondus.

9. Système pour ostéosynthèse de l'extrémité supérieure du fémur selon la revendication 8, caractérisé en ce que le moyen unique de solidarisation des deux clous (10,20), cervicocéphalique et centromédullaire est une clavette (30) qui traverse d'un part, le clou (10) centromédullaire dans sa partie supérieure par un canal axial (15), et d'autre part, le clou (20) cervicocéphalique part une lumière (27) transversale faisant un angle déterminé avec l'axe longitudinal dudit clou (20).

10. Système pour ostéosynthèse de l'extrémité supérieure du fémur selon la revendication 8, caractérisé en ce que le moyen unique de solidarisation des deux clous (10,20), cervicocéphalique et centromédullaire est une vis (70) qui traverse le clou (10) centromédullaire dans sa partie supérieure par un canal axial (15) et prend appui sur un méplat (27) pratiqué sur le clou (20) cervicocéphalique.

11. Système pour ostéosynthèse de l'extrémité supérieure du fémur selon la revendication 8, caractérisé en ce que le moyen unique de solidarisation des deux clous (10,20), cervicocéphalique et centromédullaire est une visvis (80) qui traverse le clou (10) centromédullaire dans sa partie supérieure par un canal axial (15), et vient se loger dans une cavité (29) pratiquée sur le clou (20) cervicocéphalique, la forme de la cavité (29) est troconique

12. Système pour ostéosynthèse de l'extrémité supérieure du fémur selon l'une des revendications 1 à 7, caractérisé en ce que le moyen de blocage en translation du clou (20) cervicocéphalique est une vis (70) qui prend appui sur ledit clou (20).

13. Système pour ostéosynthèse de l'extrémité supérieure du fémur selon la revendication 12, caractérisé en ce que le moyen de blocage en rotation du clou cervicocéphalique est constitué par le choix d'une forme non circulaire déterminée, pour la section du clou (20) cervicocéphalique et pour la lumière (13) transversale de la partie supérieure du clou (20) centromédullaire.

14. Système pour ostéosynthèse de l'extrémité supérieure du fémur selon l'une des revendications 1 à 13, caractérisé en ce que le moyen (11) de solidarisation du clou (10) centromédullaire avec fémur est une vis (11) corticale à verrouillage diaphisaire qui traverse lesdits clous (10) perpendiculairement à leur axe longitudinal.

15. Système pour ostéosynthèse de l'extrémité supérieure du fémur selon l'une des revendications 1 à 14, caractérisé en ce que l'angle entre l'axe de la lumière (13) transversale de la partie supérieure du clou centromédullaire et l'axe vertical (17) est compris entre 125 et 135°.

16. Système pour ostéosynthèse de l'extrémité supérieure du fémur selon l'une des revendications 1 à 15, caractérisé en ce que le dispositif de visée pour la mise en place d'un dispositif pour ostéosynthèse comporte des moyens (60,55,56) de fixation au clou (10) centromédullaire, des moyens de visée (42) pour la mise en place du clou (20) cervicocéphalique, des moyens de repérage (43) pour un positionnement optimal des moyens de blocage en rotation et en translation du clou (20) cervicocéphalique, des moyens de visée (51,52,44) pour la mise en place des moyens (26,11) de solidarisation des clous (10,20) centromédullaire et cervicocéphalique avec fémur.

17. Système pour ostéosynthèse de l'extrémité supérieure du fémur selon l'une des revendications 1 à 16, caractérisé en ce que le dispositif de visée pour la mise en place du dispositif pour ostéosynthèse comprend deux branches (40,50) fixées entre elles de façon amovible, formant un angle déterminé, une première branche (50) parallèle au clou (20) cervicocéphalique, de longueur déterminée en fonction du patient, comprenant des moyens de solidarisation (60,55,56) au clou (20) centromédullaire, des moyens (51,52) de visée pour la mise en place des moyens (26) de fixation du clou (20) cervicocéphalique sur le fémur, et une deuxième branche (40), parallèle à la partie inférieure du clou (10) centromédullaire, de longueur déterminée en fonction du patient, comportant des moyens (43) de visée pour la mise en place du clou (20) cervicocéphalique dans l'axe du col du fémur, des moyens (44) de visée pour la mise en place des moyens (11) de fixation du clou (10) centromédullaire sur le fémur, et des moyens (43) de repérage pour un positionnement optimal des moyens (28,27,30,24,70) de blocage en rotation et en translation du clou cervicocéphalique.

18. Système pour ostéosynthèse de l'extrémité supérieure du fémur selon l'une des revendications 1 à 16, caractérisé en ce que le dispositif de visée pour la mise en place du dispositif pour ostéosynthèse comprend une barre (90) coudée, la première section (91) inférieure au coude est verticale et comprend des moyens (43) de visée pour la mise en place du clou (20) cervicocéphalique dans l'axe du col du fémur, des moyens (44) de visée pour la mise en place des moyens (11) de fixation du clou (10) centromédullaire sur le fémur, et des moyens (43) de repérage pour un positionnement optimal des moyens (28,27,30,24,70) de blocage en rotation et en translation du clou cervicocéphalique, la deuxième section (92) étant sensiblement parallèle au clou (20) cervicocéphalique lorsque celui-ci est en place dans le col de fémur et comprend des moyens de solidarisation (60,55,56) au clou (20) centromédullaire, des moyens (51,52) de visée pour la mise en place des moyens (26) de fixation du clou (20) cervicocéphalique sur le fémur.

19. Système pour ostéosynthèse de l'extrémité supérieure du fémur selon la revendications 16 ou 17, caractérisé en ce que les moyens de solidarisation () comprennent une douille (60), une lumière (56) pratiquée dans la partie supérieure du système de visée, et un moyen (55) de fixation, de type boulon passant par la lumière (56), traversant la douille (60) pour coopérer avec le taraudage (14) du clou (10) centromédullaire.

20. Système pour ostéosynthèse de l'extrémité supérieure du fémur selon la revendications 16 ou 18, caractérisé en ce que les moyens de solidarisation, comprennent une pièce (100) en L, fixée par sa première branche (101), parallèle à la section (92) supérieure du système de visée, sur cette même section (92), grâce à des moyens de fixation (102), au clou (10) centromédullaire et par sa deuxième branche (103), comprenant à son l'extrémité libre une pièce (105) cylindrique creuse, sur le clou (10) centromédullaire, par l'intermédiaire d'un moyen de fixation qui traverse la pièce (105) cylindrique creuse.

21. Système pour ostéosynthèse de l'extrémité supérieure du fémur selon la revendications 16 à 20, caractérisé en ce que les moyens de visée pour la mise en place du clou (20) cervicocéphalique sont une lumière (42) transversale faisant un angle déterminé avec l'axe longitudinal (41) de la branche inférieure (40) du dispositif de visée.

22. Système pour ostéosynthèse de l'extrémité supérieure du fémur selon l'une des revendications 21, caractérisé en ce que l'angle entre la lumière (42) transversale et l'axe longitudinal de la branche inférieure (40) du dispositif de visée est le même que angle entre l'axe de la lumière (13) transversale de la partie supérieure du clou (10) centromédullaire et l'axe vertical (17).

23. Système pour ostéosynthèse de l'extrémité supérieure du fémur selon l'une des revendications 16 à 22, caractérisé en ce que la surface extérieure de la branche inférieure (40) du dispositif de visée, comporte au niveau de la lumière transversale (42), un repère (43) visualisant l'orientation que devront prendre les moyens (28,27,30,24,70) de blocage en rotation et en translation, du clou (20) cervicocéphalique, lors de la mise en place.

24. Système pour ostéosynthèse de l'extrémité supérieure du fémur selon l'une des revendications 16 à 23, caractérisé en ce que les moyens de visée pour les mises en place des moyens de fixation des deux clous (10,20) centromédullaire et cervicocéphalique, avec le fémur sont des lumières (44,51,52) perpendiculaires aux axes longitudinaux des branches (40,50) correspondantes, du dispositif de visée.
